# EUROPEAN PATENT APPLICATION

(11) **EP 1 122 318 A2**
(43) Date of publication of application: **08.08.2001**
(21) Application number: 01300807.3
(22) Date of filing: 30.01.2001
(51) Int. Cl.: C12Q 1/68

(54) **Diagnostic method for the detection of polymorphisms related to the human urokinase plasminogen activator receptor**

(30) Priority: 03.02.2000 GB 0002366
(71) Applicant: AstraZeneca AB, 151 85 Södertälje (SE)
(72) Inventor: Smith, John Craig AstraZeneca, Macclesfield, Cheshire SK10 4TG (GB)
(74) Representative: Gainey, Laurence David Scott

(57) **Abstract**

This invention relates to single nucleotide polymorphisms in the human urokinase plasminogen activator receptor (uPAR) gene and corresponding novel allelic polypeptides encoded thereby. The invention also relates to methods and materials for analysing allelic variation in the uPAR gene and to the use of said polymorphism in the diagnosis and treatment of uPAR ligand mediated diseases, such as cancer or arthritis.

### Annex to the application documents - subsequently filed sequences listing

SEQUENCE LISTING

<110> SMITH, JOHN C
      ASTRAZENECA AB

<120> DIAGNOSTIC METHOD

<130> LDSG/Z70655

<140>
<141>

<150> GB 0002366.3
<151> 2000-02-03

<160> 22

<170> PatentIn Ver. 2.1

<210> 1
<211> 31
<212> DNA
<213> Human

<400> 1
agctgggggc acagcaggaa gcaaagcaag g                                31

<210> 2
<211> 31
<212> DNA
<213> Human

<400> 2
agggaagttt gtggcggagg aggttcgtac g                                31

<210> 3
<211> 31
<212> DNA
<213> Human

<400> 3
gaaaaagaca gagttggact caaataacag a                                31

<210> 4
<211> 31

<212> DNA
<213> Human

<400> 4
cagggcaact ctggtgagta gggcagccct t                                    31

<210> 5
<211> 31
<212> DNA
<213> Human

<400> 5
agtgttacag ctgcaagggg aacagcaccc a                                    31

<210> 6
<211> 31
<212> DNA
<213> Human

<400> 6
aagaggctgt gcaaccgcct caatgtgcca a                                    31

<210> 7
<211> 31
<212> DNA
<213> Human

<400> 7
ctgcccatct cagcctcacc atcaccctgc t                                    31

<210> 8
<211> 31
<212> DNA
<213> Human

<400> 8
tggctggatc cgggggaccc ctttgccctt c                                    31

<210> 9
<211> 25
<212> DNA
<213> Human

<400> 9
tggtccagga gctgggggca cagcg                                          25

<210> 10
<211> 35
<212> DNA
<213> Human

<400> 10
gtgctgggca ctggtccagg agctgggggc actgc                               35

<210> 11
<211> 35
<212> DNA
<213> Human

<400> 11
cagccggccg cgccccggga agggaagttt gctgc                               35

<210> 12
<211> 35
<212> DNA
<213> Human

<400> 12
tggaggcaag gttaactcta gaaaaagaca gaatt                               35

<210> 13
<211> 35
<212> DNA
<213> Human

<400> 13
aaaaaccaaa gctatatggt aagaggctgt gcagc                               35

<210> 14
<211> 35
<212> DNA
<213> Human

<400> 14
ggctgctcct cagcctggcc ctgcccatct aggcc                               35

<210> 15
<211> 31
<212> DNA
<213> Human

<400> 15
agctgggggc acagcgggaa gcaaagcaag g                                    31

<210> 16
<211> 31
<212> DNA
<213> Human

<400> 16
agggaagttt gtggcagagg aggttcgtac g                                    31

<210> 17
<211> 31
<212> DNA
<213> Human

<400> 17
gaaaaagaca gagttcgact caaataacag a                                    31

<210> 18
<211> 31
<212> DNA
<213> Human

<400> 18
cagggcaact ctggtaagta gggcagccct t                                    31

<210> 19
<211> 31
<212> DNA
<213> Human

<400> 19
agtgttacag ctgcaggggg aacagcaccc a                                    31

<210> 20
<211> 31

<212> DNA
<213> Human

<400> 20
aagaggctgt gcaactgcct caatgtgcca a                                    31

<210> 21
<211> 31
<212> DNA
<213> Human

<400> 21
ctgcccatct cagccccacc atcaccctgc t                                    31

<210> 22
<211> 31
<212> DNA
<213> Human

<400> 22
tggctggatc cggggaaccc ctttgccctt c                                    31

## Description

This invention relates to single nucleotide polymorphisms in the human urokinase plasminogen activator receptor (uPAR) gene and corresponding novel allelic polypeptides encoded thereby. The invention also relates to methods and materials for analysing allelic variation in the uPAR gene and to the use of said polymorphism in the diagnosis and treatment of uPAR ligand mediated diseases, such as cancer or arthritis.

The urokinase plasminogen activator receptor (uPAR) plays a key role in cancer cell invasion and metastasis, controlling cell motility, tissue remodelling and the bioavailability of angiogenic factors. Formation of a uPA/uPAR complex at the cell surface is necessary for efficient activation of plasmin, a broad range protease which can degrade components of the basement membrane and the extracellular matrix. This degradation is a prerequisite for tumour cell invasion (Ellis *et al*., (1991) J Biol Chem. **266**:12752-12758). The distribution of uPAR concentrates uPA at the leading edge of migrating cells, generating a fully activated proteolytic cascade (Estreicher *et al.,* (1990) J Cell Biol. **111(2)**:783-792). Epidemiological studies support the targeting of uPAR for cancer therapy since high level expression of uPA and uPAR is correlated with a poor prognosis in a number of malignancies.

uPA binds to uPAR through the EGF like domain (aal-46) of the amino terminal fragment (ATF, aa 1-135). Preclinical data indicate that ATF based antagonists of uPAR could have potent anticancer activity (Crowley *et al.,* (1993) Proc Natl Acad Sci. USA. **90**:5021-5025; Kobayashi *et al*., (1994) Int J Cancer. **57**:727-733).

Intratumoural injection of a recombinant adenovirus expressing the enzymatically inactive uPA ATF has been shown to result in arrest of primary tumour growth, inhibition of metastasis and inhibition of angiogenesis in pre-established xenografts (Li *et al.,* (1998) Gene Therapy. **5**:1105-1113). A low molecular weight uPAR antagonist may be expected to have anti-invasive and anti-tumour properties (Min *et al.,* (1996) Cancer Research. **56**:2428-2433).

The uPAR gene has been localised to a region of chromosome 19q13 which has been shown to be amplified in pancreatic adenocarcinoma cell lines (Curtis *et al.,* (1998) Genomics **53**:42-55).

The mature protein (uPAR1, 283 aa) is located in the membrane by a glycolipid anchor (Casey *et al.,* (1994) Blood. **84**:1151-1156), a variant (uPAR2) is generated by alternative splicing and is thought to encode a soluble form of the receptor (Pyke *et al*., (1993) FEBS Letters. **326**:69-74).

The intron-exon boundaries of the human uPAR gene have been determined and the complete gene sequence is contained within a single cosmid clone (R28316, EMBL Accession Number AC006953, 38727 bp). Unless stated otherwise or apparent from the context, all positions herein relate to the reverse complement of the sequence positions contained within EMBL Accession Number AC006953.

Polymorphism refers to the occurrence of two or more genetically determined alternative alleles or sequences within a population. A polymorphic marker is the site at which divergence occurs. Preferably markers have at least two alleles, each occurring at frequency of greater than 1%, and more preferably at least 10%, 15%, 20%, 30% or more of a selected population.

Single nucleotide polymorphisms (SNP) are generally, as the name implies, single nucleotide or point variations that exist in the nucleic acid sequence of some members of a species. Such polymorphism variation within the species are generally regarded to be the result of spontaneous mutation throughout evolution. The mutated and normal sequences coexist within the species' population sometimes in a stable or quasi-stable equilibrium. At other times the mutation may confer some advantage to the species and with time may be incorporated into the genomes of all members of the species.

Some SNPs occur in the protein coding sequences, in which case, one of the polymorphic protein forms may possess a different amino acid which may give rise to the expression of a variant protein and, potentially, a genetic disease. Recently, it has been reported that even polymorphisms that do not result in an amino acid change can cause different structural folds of mRNA with potentially different biological functions (Shen *et al*., (1999) Proc Natl Acad Sci USA **96**:7871-7876).

The use of knowledge of polymorphisms to help identify patients most suited to therapy with particular pharmaceutical agents is often termed "pharmacogenetics". Pharmacogenetics can also be used in pharmaceutical research to assist the drug selection process. Polymorphisms are used in mapping the human genome and to elucidate the genetic component of diseases. The reader is directed to the following references for background details on pharmacogenetics and other uses of polymorphism detection: Linder *et al.* (1997), Clinical Chemistry, **43,** 254; Marshall (1997), Nature Biotechnology, **15**, 1249; International Patent Application WO 97/40462, Spectra Biomedical; and Schafer *et al.* (1998), Nature Biotechnology, **16**, 33.

A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each SNP and n is the number of SNPs. One approach to identifying mutations or polymorphisms which are correlated with clinical response is to carry out an association study using all the haplotypes that can be identified in the population of interest. The frequency of each haplotype is limited by the frequency of its rarest allele, so that SNPs with low frequency alleles are particularly useful as markers of low frequency haplotypes. As particular mutations or polymorphisms associated with certain clinical features, such as adverse or abnormal events, are likely to be of low frequency within the population, low frequency SNPs may be particularly useful in identifying these mutations (for examples see: Linkage disequilibrium at the cystathionine beta synthase (CBS) locus and the association between genetic variation at the CBS locus and plasma levels of homocysteine. *Ann Hum Genet* (1998) 62:481-90, De Stefano V, Dekou V, Nicaud V, Chasse JF, London J, Stansbie D, Humphries SE, and Gudnason V; and Variation at the von willebrand factor (vWF) gene locus is associated with plasma vWF:Ag levels:
identification of three novel single nucleotide polymorphisms in the vWF gene promoter. *Blood* (1999) 93:4277-83, Keightley AM, Lam YM, Brady JN, Cameron CL, Lillicrap D).

Clinical trials have shown that patient response to treatment with pharmaceuticals is often heterogeneous. Thus there is a need for improved approaches to pharmaceutical agent design and therapy.

Point mutations in polypeptides will be referred to as follows: natural amino acid (using 1 or 3 letter nomenclature) , position, new amino acid. For (a hypothetical) example, "D25K" or "Asp25Lys" means that at position 25 an aspartic acid (D) has been changed to lysine (K). Multiple mutations in one polypeptide will be shown between square brackets with individual mutations separated by commas.

The present invention is based on the discovery of single nucleotide polymorphisms (SNPs) in the uPAR gene. As defined herein, the uPAR gene includes exon coding sequence, intron sequences intervening the exon sequences and, 3' and 5' untranslated region (3' UTR and 5' UTR) sequences, including the promoter element of the uPAR gene.

For the avoidance of doubt the location of each of the polymorphisms (emboldened; only the most common allele illustrated) and sequence immediately flanking each polymorphism site is as follows:
a) (position 14935, 5' UTR polymorphism) 
AGCTGGGGGCACAGCAGGAAGCAAAGCAAGG
14920 14950
(SEQ ID No. 1)
b) (position 15282, 5' UTR polymorphism)
AGGGAAGTTTGTGGCGGAGGAGGTTCGTACG
15267 15297
(SEQ ID No. 2)
c) (position 19985, intron polymorphism)
GAAAAAGACAGAGTTGGACTCAAATAACAGA
19970 20000
(SEQ ID No. 3)
d) (position 20258, intron polymorphism)
CAGGGCAACTCTGGTGAGTAGGGCAGCCCTT
20243 20273
(SEQ ID No. 4)
e) (position 33251, exon 6 polymorphism)
AGTGTTACAGCTGCAAGGGGAACAGCACCCA
33236 33266
(SEQ ID No. 5)
f) (position 36468, exon 7 polymorphism)
AAGAGGCTGTGCAACCGCCTCAATGTGCCAA
36453 36483
(SEQ ID No. 6)
g) (position 36623, exon 7 polymorphism)
CTGCCCATCTCAGCCTCACCATCACCCTGCT
 
36608 36638
(SEQ ID No. 7)
h) (position 36720, 3' UTR polymorphism)
TGGCTGGATCCGGGGGACCCCTTTGCCCTTC
36705 36735
(SEQ ID No. 8)
 Each of the above-mentioned SNPs is shown with 15 nucleotides either side thereof. Using conventional alignment analysis, the person skilled in the art will be able to precisely locate the position of these SNP's within the published sequence of the uPAR gene.

According to one aspect of the present invention there is provided a method for the diagnosis of a polymorphism in uPAR in a human, which method comprises determining the sequence of the nucleic acid of the human at one or more of positions:
14935, 15282, 19985, 20258, 33251, 36468, 36623 and 36720, each defined by the position of the reverse complement of EMBL Accession Number AC006953, and determining the status of the human by reference to polymorphism in the uPAR gene.

Position 14935 is located within the promoter region of the uPAR gene and position 15282 is located within the 5' UTR of the uPAR mRNA. Position 19985 occurs within the intron sequence 5' to exon 3. Position 20258 occurs within the intron sequence 3' to exon 3, adjacent to the splice site. Position 33251 is located in exon 6 of the uPAR gene and polymorphic variation at this position alters Lys 198 (AAG) to Arg 198 (AGG). Position 36468 occurs in exon 7 and polymorphic variation at this position alters the third base of the codon encoding Thr 243 (AC**C/T**). Position 36623 occurs in exon 7 and polymorphic variation at this position results in a Leu 295 to Pro 295 transition. Position 36720 occurs within the 3' UTR.

The polymorphisms at positions 14935, 33251 and 36720 are of particular interest. The former because it creates a natural RFLP recognition site, modifies a potential transcription factor binding site and occurs at relatively high frequency (25%). Position 33251 because it results in an amino acid substitution (K198R) within a functional domain (domain III) of the protein and the latter because it also results in an amino acid substitution (L295P).

Each of the above polymorphisms are single nucleotide polymorphisms (SNPs).

Thus, in another preferred embodiment of the invention the method for diagnosis described herein is one in which the polymorphism in the uPAR protein is any one of the following: Lys198Arg and Leu295Pro.

The term human includes both a human having or suspected of having a uPAR ligand mediated disease and an asymptomatic human who may be tested for predisposition or susceptibility to such disease. At each position the human may be homozygous for an allele or the human may be a heterozygote.

In one embodiment of the invention preferably the method for diagnosis described herein is one in which the single nucleotide polymorphism at position 14935 in the 5' UTR is presence of A and/or G.

In another embodiment of the invention preferably the method for diagnosis described herein is one in which the single nucleotide polymorphism at position 15282 in the 5' UTR is presence of G and/or A.

In another embodiment of the invention preferably the method for diagnosis described herein is one in which the single nucleotide polymorphism at position 19985 near exon 3 is presence of G and/or C.

In another embodiment of the invention preferably the method for diagnosis described herein is one in which the single nucleotide polymorphism at position 20258 near exon 3 is presence of G and/or A.

In another embodiment of the invention preferably the method for diagnosis described herein is one in which the single nucleotide polymorphism at position 33251 in exon 6 is presence of A and/or G.

In another embodiment of the invention preferably the method for diagnosis described herein is one in which the single nucleotide polymorphism at position 36468 in the exon 7 is the presence of C and/or T.

In another embodiment of the invention preferably the method for diagnosis described herein is one in which the single nucleotide polymorphism at position 36623 in exon 7 is presence of T and/or C.

In another embodiment of the invention preferably the method for diagnosis described herein is one in which the single nucleotide polymorphism at position 36720 in the 3'UTR is presence of G and/or A.

Each of the above is defined by the position of the reverse complement of EMBL Accession Number AC006953.

According to another aspect of the present invention there is provided a method for the diagnosis of a single nucleotide polymorphism in uPAR in a human, which method comprises determining the sequence of the nucleic acid of the human corresponding to position 16 of one or more of SEQ ID NO: 1, 2, 3, 4, 5, 6, 7 or 8, and determining the status of the human by reference to polymorphism in the uPAR gene.

In another embodiment of the invention preferably the method for diagnosis described herein is one selected from the group in which: the single nucleotide polymorphism at position 16 according to the position in SEQ ID NO:1 is presence of A and/or G, the single nucleotide polymorphism at position 16 according to the position in SEQ ID NO:2 is presence of G and/or A, the single nucleotide polymorphism at position 16 according to the position in SEQ ID NO:3 is presence of G and/or C, the single nucleotide polymorphism at position 16 according to the position in SEQ ID NO:4 is presence of G and/or A, the single nucleotide polymorphism at position 16 according to the position in SEQ ID NO:5 is presence of A and/or G, the single nucleotide polymorphism at position 16 according to the position in SEQ ID NO:6 is presence of C and/or T, the single nucleotide polymorphism at position 16 according to the position in SEQ ID NO:7 is presence of T and/or C and, the single nucleotide polymorphism at position 16 according to the position in SEQ ID NO:8 is presence of G and/or A.

The method for diagnosis is preferably one in which the sequence is determined by a method selected from amplification refractory mutation system (ARMS™-allele specific amplification), allele specific hybridisation (ASH), oligonucleotide ligation assay (OLA) and restriction fragment length polymorphism (RFLP). The amino acid sequence method for diagnosis is preferably one which is determined by immunological methods such as enzyme linked immunosorbent assay (ELISA).

In another aspect of the invention there is provided a method of analysing a nucleic acid, comprising: obtaining a nucleic acid from an individual; and determining the base occupying any one of the following polymorphic sites: 14935, 15282, 19985, 20258, 33251, 36468, 36623 and 36720, each defined by the position of the reverse complement of EMBL Accession Number AC006953.

In another aspect of the invention there is provided a method for the diagnosis of uPAR ligand-mediated disease, which method comprises:
i) obtaining sample nucleic acid from an individual;
ii) detecting the presence or absence of a variant nucleotide at one or more of positions:
   14935, 15282, 19985, 20258, 33251, 36468, 36623 and 36720, each defined by the
   position of the reverse complement of EMBL Accession Number AC006953; and,
iii) determining the status of the human by reference to polymorphism in uPAR.

In another aspect of the invention there is provided a method for the diagnosis of uPAR ligand-mediated disease, which method comprises:
i) obtaining a protein containing sample from an individual;
ii) detecting the presence or absence of a variant uPAR polypeptide on the basis of the presence of a polymorphic amino acid at either or both amino acid positions: 198 and 295; and,
iii) determining the status of the human by reference to the presence or absence of a polymorphism in uPAR.
In a preferred embodiment the polymorphic amino acid at position 198 is presence of arginine and at position 295 is presence of proline.

The status of the human may be determined by reference to allelic variation at any one, two, three, four, five, six, seven or all eight positions. The status of the human may also be determined by one or more of the specific polymorphisms identified herein in combination with one or more other SNP's.

The following are representative examples of disclosures of uPAR antagonists:
EP-A-792647 (Boehringer Mannheim), DE-A-19635352 (Boehringer Mannheim), WO 9906387 (Hoffinan La Roche) and WO 9640747 (Chiron).

It is believed that a uPA receptor antagonist will have some clinical utility, particularly as anti-angiogenic agents useful, for example, in diabetic retinopathy, corneal angiogenesis, Kaposi's syndrome, metastasis and invasion by tumour cells and chronic inflammation (arthritis, emphysema and the like). Other therapeutic opportunities for uPAR antagonist exist in treating bone disorders and high blood glucose levels.

The test sample of nucleic acid is conveniently a sample of blood, bronchoalveolar lavage fluid, sputum, urine or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on the polymerase chain reaction (PCR). These may be used in combination with a number of signal generation systems, a selection of which is listed in Table 2. Further amplification techniques are listed in Table 3. Many current methods for the detection of allelic variation are reviewed by Nollau *et al*., Clin. Chem. **43**, 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

| **Abbreviations:** | |
|---|---|
| ALEX™ | Amplification refractory mutation system linear extension |
| APEX | Arrayed primer extension |
| ARMS™ | Amplification refractory mutation system |
| b-DNA | Branched DNA |
| CMC | Chemical mismatch cleavage |
| bp | base pair |
| COPS | Competitive oligonucleotide priming system |
| DGGE | Denaturing gradient gel electrophoresis |
| FRET | Fluorescence resonance energy transfer |
| LCR | Ligase chain reaction |
| MASDA | Multiple allele specific diagnostic assay |
| NASBA | Nucleic acid sequence based amplification |
| uPAR | urokinase Plasminogen Activator Receptor |
| OLA | Oligonucleotide ligation assay |
| PCR | Polymerase chain reaction |
| PTT | Protein truncation test |
| RFLP | Restriction fragment length polymorphism |
| SDA | Strand displacement amplification |
| SERRS | Surface enhanced raman resonance spectroscopy |
| SNP | Single nucleotide polymorphism |
| SSCP | Single-strand conformation polymorphism analysis |
| SSR | Self sustained replication |
| TGGE | Temperature gradient gel electrophoresis |
| 3' UTR | 3' untranslated region |

### Table 1 - Mutation Detection Techniques

**General**: DNA sequencing, Sequencing by hybridisation
**Scanning:** PTT*, SSCP, DGGE, TGGE, Cleavase, Heteroduplex analysis, CMC, Enzymatic mismatch cleavage
^{*} Note: not useful for detection of promoter polymorphisms.
**Hybridisation Based**

### Solid phase hybridisation: Dot blots, MASDA, Reverse dot blots, Oligonucleotide arrays (DNA Chips)

Solution phase hybridisation: Taqman™ - US-5210015 & US-5487972 (Hoffmann-La Roche), Molecular Beacons - Tyagi *et al* (1996), Nature Biotechnology, **14**, 303; WO 95/13399 (Public Health Inst., New York)
**Extension Based:** ARMS™-allele specific amplification (as described in European patent No. EP-B-332435 and US patent No. 5,595,890), ALEX™ - European Patent No. EP 332435 B1 (Zeneca Limited), COPS - Gibbs *et al* (1989), Nucleic Acids Research, **17**, 2347. **Incorporation Based:** Mini-sequencing, APEX
**Restriction Enzyme Based:** RFLP, Restriction site generating PCR
**Ligation Based:** OLA
**Other:** Invader assay

### Table 2 - Signal Generation or Detection Systems

**Fluorescence**: FRET, Fluorescence quenching, Fluorescence polarisation - United Kingdom Patent No. 2228998 (Zeneca Limited)
**Other:** Chemiluminescence, Electrochemiluminescence, Raman, Radioactivity, Colorimetric, Hybridisation protection assay, Mass spectrometry, SERRS - WO 97/05280 (University of Strathclyde).

### Table 3 - Further Amplification Methods

SSR, NASBA, LCR, SDA, b-DNA

Preferred mutation detection techniques include ARMS™-allele specific amplification, ALEX™, COPS, Taqman, Molecular Beacons, RFLP, OLA, restriction site based PCR and FRET techniques.

Particularly preferred methods include ARMS™-allele specific amplification, OLA and RFLP based methods. ARMS™-allele specific amplification is an especially preferred method.

ARMS™-allele specific amplification (described in European patent No. EP-B-332435, US patent No. 5,595,890 and Newton et al. (Nucleic Acids Research, Vol. 17, p.2503; 1989)), relies on the complementarity of the 3' terminal nucleotide of the primer and its template.
The 3' terminal nucleotide of the primer being either complementary or non-complementary to the specific mutation, allele or polymorphism to be detected. There is a selective advantage for primer extension from the primer whose 3' terminal nucleotide complements the base mutation, allele or polymorphism. Those primers which have a 3' terminal mismatch with the template sequence severely inhibit or prevent enzymatic primer extension. Polymerase chain reaction or unidirectional primer extension reactions therefore result in product amplification when the 3' terminal nucleotide of the primer complements that of the template, but not, or at least not efficiently, when the 3' terminal nucleotide does not complement that of the template.

By way of representative example, a suitable allele specific primer (ARMS primer) capable of detecting/diagnosing the "14935" polymorphism in the 5'UTR is:
5'-TGGTCCAGGAGCTGGGGGCACAGC**G**-3' (SEQ ID No. 9). The 3' terminal nucleotide complementing the "C" polymorphism on the anti-sense template strand facilitates efficient primer extension with the suitable enzyme (preferably one lacking 3'-5' exonuclease activity).

In a further aspect, the diagnostic methods of the invention are used to assess the efficacy of therapeutic compounds in the treatment of uPAR ligand mediated diseases, such as cancer, rheumatoid arthritis and other angiogenic or inflammatory associated diseases.

The polymorphisms identified in the present invention that occur in intron regions or in the promoter region are not expected to alter the amino acid sequence of the uPA receptor, but may affect the transcription and/or message stability of the sequences and thus affect the level of the receptors in cells.

Assays, for example reporter-based assays, may be devised to detect whether one or more of the above polymorphisms affect transcription levels and/or message stability.

Individuals who carry particular allelic variants of the uPAR gene may therefore exhibit differences in their ability to regulate protein biosynthesis under different physiological conditions and will display altered abilities to react to different diseases. In addition, differences in protein regulation arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition and/or susceptibility of an individual to diseases mediated by uPAR ligands. The present invention may be used to recognise individuals who are particularly at risk from developing these conditions.

In a further aspect, the diagnostic methods of the invention are used in the development of new drug therapies which selectively target one or more allelic variants of the uPAR gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes. For example the polymorphism at position 33251 can be detected by digestion with the restriction enzyme Pst 1, as polymorphism at this position modifies a Pst I site (CTGCA**A/G**).

Engineered sites include those wherein the primer sequences employed to amplify the target sequence participates along with the nucleotide polymorphism to create a restriction site For example, the polymorphism at position 14935 can be detected by diagnostic engineered RFLP digestion with the restriction enzyme Pst 1, since modification of position 14932 creates a Pst I site (CTGCAG). Polymorphism at position 14935 will modify the recognition sequence (CTGC**A/G**G).

The person of ordinary skill will be able to design and implement diagnostic procedures based on the detection of restriction fragment length polymorphism due to the loss or gain of one or more of the sites.

According to another aspect of the present invention there is provided a nucleic acid comprising any one of the following polymorphisms:
the nucleic acid sequence of EMBL Accession Number AC006953 with G at position 14935 in the promoter region as defined by the position of the reverse complement of AC006953;
the nucleic acid sequence of EMBL Accession Number AC006953 with A at position 15282 in the 5' UTR as defined by the position of the reverse complement of AC006953;
the nucleic acid sequence of EMBL Accession Number AC006953 with C at position 19985 near exon 3 as defined by the position of the reverse complement of AC006953;
the nucleic acid sequence of EMBL Accession Number AC006953 with A at position 20258 near exon 3 as defined by the position of the reverse complement of AC006953;
the nucleic acid sequence of EMBL Accession Number AC006953 with G at position 33251 in exon 6 as defined by the position of the reverse complement of AC006953;
the nucleic acid sequence of EMBL Accession Number AC006953 with T at position 36468 in exon 7 as defined by the position of the reverse complement of AC006953;
the nucleic acid sequence of EMBL Accession Number AC006953 with C at position 36623 in exon 7 as defined by the position of the reverse complement of AC006953; and,
the nucleic acid sequence of EMBL Accession Number AC006953 with A at position 36720 in the 3' UTR as defined by the position of the reverse complement of AC006953,
or a complementary strand thereof or a fragment thereof of at least 17 bases comprising at least one of the polymorphisms.

According to another aspect of the present invention there is provided an isolated nucleic acid comprising any one of the following polymorphism containing sequences: the nucleic acid sequence of SEQ ID NO:1 with G at position 16; the nucleic acid sequence of SEQ ID NO:2 with A at position 16; the nucleic acid sequence of SEQ ID NO:3 with C at position 16; the nucleic acid sequence of SEQ ID NO:4 with A at position 16; the nucleic acid sequence of SEQ ID NO:5 with G at position 16; the nucleic acid sequence of SEQ ID NO:6 with T at position 16; the nucleic acid sequence of SEQ ID NO:7 with C at position 16; the nucleic acid sequence of SEQ ID NO:8 with A at position 16, or a complementary strand thereof.

According to another aspect of the present invention there is provided an isolated nucleic acid comprising at least 17 consecutive bases of uPAR gene said nucleic acid comprising one or more of the following polymorphic alleles: G at position 14935, A at position 15282, C at position 19985, A at position 20258, G at position 33251, T at position 36468, C at position 36623 and A at position 36720, or a complementary strand thereof.

Fragments are at least 17 bases, more preferably at least 20 bases, more preferably at least 30 bases.

The invention further provides nucleotide primers which can detect the polymorphisms of the invention.

According to another aspect of the present invention there is provided an allele specific primer capable of detecting a uPAR gene polymorphism of the invention.

An allele specific primer is used, generally together with a constant primer, in an amplification reaction such as a PCR reaction, which provides the discrimination between alleles through selective amplification of one allele at a particular sequence position e.g. as used for ARMS™ assays. The allele specific primer is preferably 17- 50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer. Often the nucleotide at the -2 and/or -3 position (relative to the 3' terminus) is mismatched in order to optimise differential primer binding and preferential extension from the correct allele discriminatory primer only.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting a uPAR gene polymorphism of the invention.

According to another aspect of the present invention there is provided an allele-specific oligonucleotide probe capable of detecting an uPAR gene polymorphism at one or more of positions: 14935, 15282, 19985, 20258, 33251, 36468, 36623 and 36720, each defined by the position of the reverse complement of EMBL Accession Number AC006953, in the uPAR gene.

The allele-specific oligonucleotide probe is preferably 17-50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. Suitable oligonucleotide probes might be those consisting of or comprising the sequences depicted in SEQ ID Nos. 1 - 8 or 15-22, or sequences complementary thereto. The central emboldened nucleotide (the polymorphism site) as illustrated in these SEQ ID's (see above) could be altered to ensure specific hybridisation to, and thus detection of, an alternate variant allele. The probes of the invention may carry one or more labels to facilitate detection, such as in Molecular Beacons.

According to another aspect of the present invention there is provided a diagnostic kit comprising one or more diagnostic probe(s) of the invention and/or diagnostic primer(s), particularly an allele-specific oligonucleotide primer, of the invention.

The diagnostic kits may comprise appropriate packaging and instructions for use in the methods of the invention. Such kits may further comprise appropriate buffer(s) and polymerase(s) such as thermostable polymerases, for example taq polymerase. Such kits may also comprise companion/constant primers and/or control primers or probes. A companion/constant primer is one that is part of the pair of primers used to perform PCR. Such primer usually complements the template strand precisely.

In another aspect of the invention, the single nucleotide polymorphisms of this invention may be used as genetic markers in linkage studies. This particularly applies to the polymorphism in the promoter region (position 14935) because of its relatively high frequency (see below). Polymorphisms that occur relatively infrequently are useful as markers of low frequency haplotypes.

According to another aspect of the present invention there is provided a method of treating a human in need of treatment with a uPAR ligand antagonist drug in which the method comprises:
i) diagnosis of a single nucleotide polymorphism in uPAR gene in the human, which diagnosis comprises determining the sequence of the nucleic acid at one or more of positions: 14935, 15282, 19985, 20258, 33251, 36468, 36623 and 36720, each defined by the position of the reverse complement of EMBL Accession Number AC006953;
ii) determining the status of the human by reference to polymorphism in the uPAR gene; and
iii) administering an effective amount of a uPAR ligand antagonist.

According to another aspect of the present invention there is provided a method of treating a human in need of treatment with a uPAR ligand antagonist drug in which the method comprises:
i) diagnosis of a polymorphism in uPAR protein in the human, which diagnosis comprises determining the amino acid one or both of positions:198 and 295 of the uPAR protein;
ii) determining the status of the human by reference to polymorphism in the uPAR protein; and,
iii) administering an effective amount of a uPAR ligand antagonist.

Preferably determination of the status of the human is clinically useful. Examples of clinical usefulness include deciding which antagonist drug of drugs to administer and/or in deciding on the effective amount of the drug or drugs.

According to another aspect of the present invention there is provided use of an uPAR ligand antagonist drug in preparation of a medicament for treating a uPAR ligand mediated disease in a human diagnosed as having a particular single nucleotide polymorphism at one or more of positions: 14935, 15282, 19985, 20258, 33251, 36468, 36623 and 36720, each defined by the position of the reverse complement of EMBL Accession Number AC006953.

According to another aspect of the present invention there is provided a pharmaceutical pack comprising an uPAR antagonist drug and instructions for administration of the drug to humans diagnostically tested for a single nucleotide polymorphism at one or more of positions: 14935, 15282, 19985, 20258, 33251, 36468, 36623 and 36720, each defined by the position of the reverse complement of EMBL Accession Number AC006953.

Testing for the presence of the polymorphism at position 33251 in exon 6 and/or position 36623 in exon 7 is especially preferred because, without wishing to be bound by theoretical considerations, of their resulting in a significant amino acid change in uPAR polypeptide (as explained herein).

The nucleic acid sequences of the invention, particularly those relating to and identifying the single nucleotide polymorphisms identified herein represent a valuable information source with which to identify further sequences of similar identity and characterise individuals in terms of, for example, their identity, haplotype and other sub-groupings, such as susceptibility to treatment with particular drugs. These approaches are most easily facilitated by storing the sequence information in a computer readable medium and then using the information in standard macromolecular structure programs or to search sequence databases using state of the art searching tools such as GCG (Genetics Computer Group), BlastX, BlastP, BlastN, FASTA (refer to Altschul *et al.* (1990) J. Mol. Biol. **215**:403-410). Thus, the nucleic acid sequences of the invention are particularly useful as components in databases useful for sequence identity, genome mapping, pharmacogenetics and other search analyses. Generally, the sequence information relating to the nucleic acid sequences and polymorphisms of the invention may be reduced to, converted into or stored in a tangible medium, such as a computer disk, preferably in a computer readable form. For example, chromatographic scan data or peak data, photographic scan or peak data, mass spectrographic data, sequence gel (or other) data.

The invention provides a computer readable medium having stored thereon one or more nucleic acid sequences of the invention. For example, a computer readable medium is provided comprising and having stored thereon a member selected from the group consisting of: a nucleic acid comprising the sequence of a nucleic acid of the invention, a nucleic acid consisting of a nucleic acid of the invention, a nucleic acid which comprises part of a nucleic acid of the invention, which part includes at least one of the polymorphisms of the invention, a set of nucleic acid sequences wherein the set includes at least one nucleic acid sequence of the invention, a data set comprising or consisting of a nucleic acid sequence of the invention or a part thereof comprising at least one of the polymorphisms identified herein. The computer readable medium can be any composition of matter used to store information or data, including, for example, floppy disks, tapes, chips, compact disks, digital disks, video disks, punch cards and hard drives.

According to another aspect of the invention there is provided a computer readable medium having stored thereon a nucleic acid sequence comprising at least 17, preferably at least 20 consecutive bases of the uPAR gene sequence, which sequence includes at least one of the polymorphisms at positions: 14935, 15282, 19985, 20258, 33251, 36468, 36623 and 36720, according to the position of the reverse complement of EMBL Accession Number AC006953.

According to another aspect of the invention there is provided a computer readable medium having stored thereon a nucleic acid sequence comprising at least 17, preferably at least 20 consecutive bases of the uPAR gene sequence, which sequence includes at least one of the following polymorphisms: G at position 14935, A at position 15282, C at position 19985, A at position 20258, G at position 33251, T at position 36468, C at position 36623 and A at position 36720, or a complementary strand thereof.

A computer based method is also provided for performing sequence identification, said method comprising the steps of providing a nucleic acid sequence comprising a polymorphism of the invention in a computer readable medium; and comparing said polymorphism containing nucleic acid sequence to at least one other nucleic acid or polypeptide sequence to identify identity (homology), i.e. screen for the presence of a polymorphism. Such a method is particularly useful in pharmacogenetic studies and in genome mapping studies.

In another aspect of the invention there is provided a method for performing sequence identification, said method comprising the steps of providing a nucleic acid sequence comprising at least 20 consecutive bases of the uPAR gene sequence, which sequence includes at least one of the polymorphisms at positions: 14935, 15282, 19985, 20258, 33251, 36468, 36623 and 36720 (according to the position of the reverse complement of EMBL Accession Number AC006953) in a computer readable medium; and comparing said nucleic acid sequence to at least one other nucleic acid sequence to identify identity.

In another aspect of the invention there is provided a method for performing sequence identification, said method comprising the steps of providing a nucleic acid sequence comprising a sequence selected from the group consisting of: the nucleic acid sequence of EMBL Accession Number AC006953 with G at position 14935 in the 5' UTR as defined by the position of the reverse complement of AC006953;
the nucleic acid sequence of EMBL Accession Number AC006953 with A at position 15282 in the 5' UTR as defined by the position of the reverse complement of AC006953;
the nucleic acid sequence of EMBL Accession Number AC006953 with C at position 19985 near exon 3 as defined by the position of the reverse complement of AC006953;
the nucleic acid sequence of EMBL Accession Number AC006953 with A at position 20258 near exon 3 as defined by the position of the reverse complement of AC006953;
the nucleic acid sequence of EMBL Accession Number AC006953 with G at position 33251 in exon 6 as defined by the position of the reverse complement of AC006953;
the nucleic acid sequence of EMBL Accession Number AC006953 with T at position 36468 in exon 7 as defined by the position of the reverse complement of AC006953;
the nucleic acid sequence of EMBL Accession Number AC006953 with C at position 36623 in exon 7 as defined by the position of the reverse complement of AC006953; and,
the nucleic acid sequence of EMBL Accession Number AC006953 with A at position 36720 in the 3' UTR as defined by the position of the reverse complement of AC006953;
or a complementary strand thereof or a fragment thereof of at least 17 bases comprising at least one of the polymorphisms; and comparing said nucleic acid sequence to at least one other nucleic acid or polypeptide sequence to identify identity.

The invention will now be illustrated but not limited by reference to the following Examples. All temperatures are in degrees Celsius.

In the Examples below, unless otherwise stated, the following methodology and materials have been applied.

AMPLITAQ™, available from Perkin-Elmer Cetus, is used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989).

Electropherograms were obtained in a standard manner: data was collected by ABI377 data collection software and the wave form generated by ABI Prism sequencing analysis (2.1.2).

### EXAMPLES

### Example 1 - Identification of Polymorphisms

### A. Methods

### DNA Preparation

DNA was prepared from frozen blood samples collected in EDTA following protocol I (Molecular Cloning: A Laboratory Manual, p392, Sambrook, Fritsch and Maniatis, 2^{nd} Edition, Cold Spring Harbor Press, 1989) with the following modifications. The thawed blood was diluted in an equal volume of standard saline citrate instead of phosphate buffered saline to remove lysed red blood cells. Samples were extracted with phenol, then phenol/chloroform and then chloroform rather than with three phenol extractions. The DNA was dissolved in deionised water.

### Template Preparation

Templates were prepared by PCR using the oligonucleotide primers and annealing temperatures set out below. The extension temperature was 72° and denaturation temperature 94°. Generally 50 ng of genomic DNA was used in each reaction and subjected to 35 cycles of PCR.

### Dye Primer Sequencing

Dye-primer sequencing using M13 forward and reverse primers was as described in the ABI protocol P/N 402114 for the ABI Prism™ dye primer cycle sequencing core kit with "AmpliTaq FS"™ DNA polymerase, modified in that the annealing temperature was 45° and DMSO was added to the cycle sequencing mix to a final concentration of 5 %.

The extension reactions for each base were pooled, ethanol/sodium acetate precipitated, washed and resuspended in formamide loading buffer.

4.25 % Acrylamide gels were run on an automated sequencer (ABI 377, Applied Biosystems).

### B. Results

### Primer design

Primers were designed using the sequence contained within EMBL Accession Number AC006953, numbering refers to the reverse complement of this sequence.

| | |
|---|---|
| Exon1 | 15019-15504 |
| 2 | 17880-17990 |
| 3 | 20112-20255 |
| 4 | 28931-29092 |
| 5 | 29998-30132 |
| 6 | 33200-33346 |
| 7 | 36428-36991 |

| **Product** | **Forward Primer** | **Reverse primer** | **Temp** | **Time** |
|---|---|---|---|---|
| 13759-14195 | 13759-13782 | 14172-14195 | 55o | 90 seconds |
| 14108-14577 | 14108-14130 | 14554-14577 | 55o | 90 seconds |
| 14463-14877 | 14463-14486 | 14877-14900 | 55o | 90 seconds |
| 14811-15265 | 14811-14833 | 15265-15285 | 55o | 90 seconds |
| 15102-15534 | 15102-15125 | 15534-15555 | 55o | 90 seconds |
| 17831-18095 | 17831-17854 | 18072-18095 | 55o | 90 seconds |
| 19915-20333 | 19915-19937 | 20310-20333 | 55o | 90 seconds |
| 28839-29156 | 28839-28862 | 29133-29156 | 55o | 90 seconds |
| 29956-30223 | 29956-29979 | 30201-30223 | 55o | 90 seconds |
| 33151-33423 | 33151-33174 | 33400-33423 | 55o | 90 seconds |
| 36388-36833 | 36388-36408 | 36810-36833 | 55o | 90 seconds |

For dye-primer sequencing, these primers were modified to include the M13 forward and reverse primer sequences (ABI protocol P/N 402114, Applied Biosystems) at the 5' end of the forward and reverse oligonucleotides respectively.

| **Novel Polymorphisms** | | | |
|---|---|---|---|
| **(1)** | | | |
| **Position** | **Polymorphism** | **Allele Frequency** | **No of Individuals** |
| 14935 | A/G | A 75% G 25% | 20 |

Polymorphism at position 14935 modifies a potential PEA3 transcription factor binding site (**A/G**GGAAG). The polymorphism at position 14935 can be detected by a diagnostic engineered Restriction Fragment Length Polymorphism (eRFLP), since modification of position 14932 creates a Pst I site (CTGCAG). Polymorphism at position 14935 will modify the recognition sequence (CTGC**A/G**G).

### Diagnostic primer (Positions 14900-14934 in AC006953)

GTGCTGGGCACTGGTCCAGGAGCTGGGGGCACTGC (SEQ ID No. 10)

Modified residue is shown in bold underline

### Constant primer (Positions 15264-15285 in AC006953)

Amplification of genomic DNA with these primers generates a PCR product of 285 bp. Digestion of a product generated from a wild type template with Pst I (New England Biolabs) gives rise to products of 250 bp and 35 bp. Digestion of a heterozygote product generates products of 285 bp, 250 bp and 35 bp. A homozygote variant product will not be digested by Pst I. Products can be separated and visualised on agarose gels following standard procedures (i.e. Sambrook *et al.,* 1989).

| | | | |
|---|---|---|---|
| **(2)** | | | |

| **Position** | **Polymorphism** | **Allele Frequency** | **No of Individuals** |
|---|---|---|---|
| 15282 | G/A | G 98% A 2% | 20 |

Polymorphism at position 15282 occurs within the 5' UTR of the uPAR mRNA. The polymorphism at position 15282 can be detected by a diagnostic engineered Restriction Fragment Length Polymorphism (eRFLP), since modification of positions15278, 15279 creates a potential Pst I site (CTGCAG). Polymorphism at position 15282 will modify the ) recognition sequence (CTGC**A**/**G**G).

### Diagnostic primer (Positions 15247-15281 in AC006953)

CAGCCGGCCGCGCCCCGGGAAGGAAGTTTGCTGC (SEQ ID No. 11)

Modified residues in bold underline

### Constant primer (Positions 15555-15534 in AC006953)

Amplification of genomic DNA with these primers generates a PCR product of 308 bp. A product generated from a wild type template will not be digested by Pst I (New England Biolabs). Digestion of a heterozygote product will generate products of 308 bp, 273 bp and 35 bp. Digestion of a homozygote variant will generate products of 273 bp and 35 bp. Products can be separated and visualised on agarose gels following standard procedures (i.e. Sambrook et al., 1989).

| | | | |
|---|---|---|---|
| **(3)** | | | |

| **Position** | **Polymorphism** | **Allele Frequency** | **No of Individuals** |
|---|---|---|---|
| 19985 | G/C | G 83% C 17% | 20 |

Polymorphism at position 19985 occurs in intron sequence 5' to exon 3. The polymorphism at position 19985 can be detected by a diagnostic eRFLP, since modification of position 19982 creates an Eco RI site (GAATTC). Polymorphism at position 19885 will modify the recognition sequence (GAATT**G/C**).

### Diagnostic primer (Positions 19950-19984 in AC006953)

TGGAGGCAAGGTTAACTCTAGAAAAAGACAGAATT (SEQ ID No. 12)

Modified residue in bold underline

### Constant primer (Positions 20333-20310 in AC006953)

Amplification of genomic DNA with these primers will generate a PCR product of 383 bp. A product generated from a wild type template will not be digested by Eco RI (New England Biolabs). Digestion of a heterozygote product will generate products of 383 bp, 349 bp and 34 bp. Digestion of a homozygous variant will generate products of 349 bp and 34 bp. Products can be separated and visualised on agarose gels following standard procedures (i.e. Sambrook *et al*., 1989).

| | | | |
|---|---|---|---|
| **(4)** | | | |

| **Position** | **Polymorphism** | **Allele Frequency** | **No of Individuals** |
|---|---|---|---|
| 20258 | G/A | G98% A2% | 20 |

Polymorphism at position 20258 occurs in intron sequence 3' to exon 3 and is adjacent to the splice site

### Exon 3...CTCTGgtg/a

Exon sequence upper case, intron sequence lower case.

| | | | |
|---|---|---|---|
| **(5)** | | | |

| **Position** | **Polymorphism** | **Allele Frequency** | **No of Individuals** |
|---|---|---|---|
| 33251 | A/G | A 87% G 13% | 20 |

Polymorphism at position 33251 is located in exon 6 of the uPAR gene and alters Lys 198 (AAG) to Arg 198 (AGG). Lys 198 is adjacent to a disulphide bridge in Domain III of the mature uPAR protein (Ploug and Ellis (1994) FEBS Letts. 349:163-168). The polymorphism modifies a Pst I site (CTGCA**A/G**).

| **Product** | **Forward Primer** | **Reverse primer** | **Temp** | **Time** |
|---|---|---|---|---|
| 33151-33423 | 33151-33174 | 33400-33423 | 55o | 90 seconds |

A PCR product generated from a wild type template (272 bp) will not be digested by the restriction enzyme, Pst I (New England Biolabs). Digestion of a PCR product from a heterozygote with Pst I will give rise to products of 272 bp, 172 bp and 100 bp. Digestion of a PCR product from a homozygous variant with Pst I will give rise to products of 172 bp and 100 bp. Products can be separated and visualised on agarose gels following standard procedures (i.e. Sambrook *et al.,* 1989).

| **(6)** | | | |
|---|---|---|---|
| **Position** | **Polymorphism** | **Allele Frequency** | **No of Individuals** |
| 36468 | C/T | C98%T 2% | 20 |

Polymorphism at position 36468 occurs in exon 7 and alters the third base of the codon encoding Thr 243 (AC**C/T**). It has been shown that single nucleotide polymorphisms can cause different structural folds of mRNA with potentially different biological functions (Shen et al., 1999, *ibid*). The polymorphism at position 36468 can be detected by a diagnostic e RFLP, since modification of position 36466 creates a potential Pvu II site (CAGCTG). Polymorphism at position 36468 will modify the recognition sequence (CAGC**C/T**G).

### Diagnostic primer (Positions 36433-36467 in AC006953)

AAAAACCAAAGCTATATGGTAAGAGGCTGTGCAGC (SEQ ID No. 13)

Modified residues in bold underline

### Constant primer (Positions 36810-36833 in AC006953)

Amplification of genomic DNA with these primers will generate a PCR product of 400 bp. A product generated from a wild type template will not be digested by Pvu II (New England Biolabs), digestion of a heterozygote product will give rise to products of 400 bp, 367 bp and 33 bp. Digestion of a homozygous variant product will generate products of 367 bp and 33 bp. Products can be separated and visualised on agarose gels following standard procedures (i.e. Sambrook *et al*., 1989).

| **(7)** | | | |
|---|---|---|---|
| **Position** | **Polymorphism** | **Allele Frequency** | **No of Individuals** |
| 36623 | T/C | T 85% C 15% | 20 |

Polymorphism at position 36623 occurs in exon 7 and results in a Leu 295 to Pro 295 transition. The mature protein is generated by cleavage after codon 283 (Gly) and attachment of a glycolipid anchor. It is thought that the signals for glycolipid anchor attachment are encoded within the C -terminal sequence, variation within this region may effect processing and attachment of the glycolipid and subsequent localisation of the mature protein in the cell membrane (Moeller *et al*., (1992) Eur J Biochem **208**:493-500; Aceto *et al*., (1999) Biochemistry 38:992-1001).

The polymorphism at position 36623 can be detected by a diagnostic eRFLP, since modification of positions 36618, 36619 creates a Stu I site (AGGCCT). Polymorphism at position 36623 will modify the recognition sequence (AGGCC**T/C**).

### Diagnostic primer (Positions 36588-36622 in AC006953)

GGCTGCTCCTCAGCCTGGCCCTGCCCATCTAGGCC (SEQ ID No. 14)

Modified residues in bold underline

### Constant primer (Positions 36810-36833 in AC006953)

Amplification of genomic DNA with these primers will generate a PCR product of 245 bp. Digestion of product generated from a wild type template with Stu I will give rise to products of 213 bp and 32 bp. Digestion of a heterozygote product will generate products of 245 bp, 213 bp and 32 bp. The homozygote variant product will not be cleaved by Stu I. Products can be separated and visualised on agarose gels following standard procedures (i.e. Sambrook *et al*., 1989).

| **(8)** | | | |
|---|---|---|---|
| **Position** | **Polymorphism** | **Allele Frequency** | **No of Individuals** |
| 36720 | G/A | G93% A7% | 20 |

Polymorphism at position 36720 occurs within the 3' UTR.

SEQ ID NO: 15
AGCTGGGGGCACAGCGGGAAGCAAAGCAAGG

SEQ ID No. 16
AGGGAAGTTTGTGGCAGAGGAGGTTCGTACG

SEQ ID No. 17
GAAAAAGACAGAGTTCGACTCAAATAACAGA

SEQ ID No. 18
CAGGGCAACTCTGGTAAGTAGGGCAGCCCTT

SEQ ID No. 19
AGTGTTACAGCTGCAGGGGGAACAGCACCCA

SEQ ID No. 20
AAGAGGCTGTGCAACTGCCTCAATGTGCCAA

SEQ ID No. 21
CTGCCCATCTCAGCCCCACCATCACCCTGCT

SEQ ID No. 22
TGGCTGGATCCGGGGAACCCCTTTGCCCTTC

## Claims

1. A method for the diagnosis of a polymorphism in uPAR in a human, which method comprises determining either the sequence of the nucleic acid of the human at one or more of positions: 14935, 15282, 19985, 20258, 33251, 36468, 36623 and 36720, each defined by the position of the reverse complement of EMBL Accession Number AC006953, or the sequence of the amino acid in the uPAR protein at positions 198 or 295, and determining the status of the human by reference to polymorphism in the uPAR gene or protein.

2. A method according to claim 1 in which the single nucleotide polymorphism, according to the position of the reverse complement of EMBL Accession Number AC006953, at position 14935 in the promoter region is presence of A and/or G; at position 15282 in the 5' UTR is presence of G and/or A; at position 19985 near exon 3 is presence of G and/or C; at position 20258 near exon 3 is presence of G and/or A; at position 33251 in exon 6 is presence of A and/or G; at position 36468 in the exon 7 is the presence of C and/or T; at position 36623 in exon 7 is presence of T and/or C; and, at position 36720 in the 3'UTR is presence of G and/or A.

3. A method as claimed in claim 1 or 2, wherein the nucleic acid region containing the potential single nucleotide polymorphism is amplified by polymerase chain reaction prior to determining the sequence.

4. A method as claimed in any of claims 1 - 3, wherein the presence or absence of the single nucleotide polymorphism is detected by reference to the loss or gain of, optionally engineered, sites recognised by restriction enzymes.

5. A method according to claim 1 or claim 2, in which the sequence is determined by a method selected from ARMS-allele specific amplification, allele specific hybridisation, oligonucleotide ligation assay and restriction fragment length polymorphism (RFLP).

6. A method according to claim 1 wherein the presence of a polymorphic amino acid residue in the uPAR protein is determined by immunological methods such as enzyme linked immunosorbent assay (ELISA).

7. A method as claimed in any of the preceding claims for use in assessing the predisposition and/or susceptibility of an individual to diseases mediated by uPAR ligands.

8. A method for the diagnosis of uPAR ligand-mediated disease, which method comprises:
obtaining sample nucleic acid from an individual;
detecting the presence or absence of a variant nucleotide at one or more of positions:
15282, 19985, 20258, 33251, 36468, 36623 and 36720, each defined by the position of the reverse complement of EMBL Accession Number AC006953; and,
determining the status of the human by reference to polymorphism in uPAR.

9. A method for the diagnosis of uPAR ligand-mediated disease, which method comprises:
obtaining a protein containing sample from an individual;
detecting the presence or absence of a variant uPAR polypeptide on the basis of the presence of a polymorphic amino acid at either or both amino acid positions: 198 and 295; and, determining the status of the human by reference to the presence or absence of a polymorphism in uPAR.

10. An isolated nucleic acid comprising any one of the following polymorphism containing sequences: the nucleic acid sequence of SEQ ID NO: 1 with G at position 16; the nucleic acid sequence of SEQ ID NO:2 with A at position 16; the nucleic acid sequence of SEQ ID NO:3 with C at position 16; the nucleic acid sequence of SEQ ID NO:4 with A at position 16; the nucleic acid sequence of SEQ ID NO:5 with G at position 16; the nucleic acid sequence of SEQ ID NO:6 with T at position 16; the nucleic acid sequence of SEQ ID NO:7 with C at position 16; the nucleic acid sequence of SEQ ID NO:8 with A at position 16, or a complementary strand thereof.

11. A diagnostic nucleic acid primer capable of detecting a polymorphism in the uPAR gene at one or more of positions: 14935, 15282, 19985, 20258, 33251, 36468, 36623 and 36720, each defined by the position of the reverse complement of EMBL Accession Number AC006953.

12. A diagnostic primer as claimed in claim 11 which is an allele specific primer adapted for use in ARMS.

13. An allele-specific oligonucleotide probe capable of detecting a polymorphism in the NK2R gene at one or more of positions: 14935, 15282, 19985, 20258, 33251, 36468, 36623 and 36720, each defined by the position of the reverse complement of EMBL Accession Number AC006953.

14. An allele specific nucleotide probe which comprises the sequence disclosed in any one of SEQ ID Nos: 1 - 8 or 15 - 22, or a sequence complementary thereto.

15. A diagnostic kit comprising one or more diagnostic primer(s) as defined in claim 11 or 12 and/or one or more allele-specific oligonucleotide probes(s) as defined in claim 13 or 14.

16. A method of treating a human in need of treatment with a uPAR ligand antagonist drug in which the method comprises:
diagnosis of a single nucleotide polymorphism in uPAR gene in the human, which diagnosis comprises determining the sequence of the nucleic acid at one or more of positions:14935, 15282, 19985, 20258, 33251, 36468, 36623 and 36720, each defined by the position of the reverse complement of EMBL Accession Number AC006953;
determining the status of the human by reference to polymorphism in the uPAR gene; and, administering an effective amount of a uPAR ligand antagonist.

17. A method of treating a human in need of treatment with a uPAR ligand antagonist drug in which the method comprises:
diagnosis of a polymorphism in uPAR protein in the human, which diagnosis comprises determining the amino acid one or both of positions:198 and 295 of the uPAR protein; determining the status of the human by reference to polymorphism in the uPAR protein; and, administering an effective amount of a uPAR ligand antagonist.

18. Use of an uPAR ligand antagonist drug in preparation of a medicament for treating a uPAR ligand mediated disease in a human diagnosed as having a particular single nucleotide polymorphism at one or more of positions: 14935, 15282, 19985, 20258, 33251, 36468, 36623 and 36720, each defined by the position of the reverse complement of EMBL Accession Number AC006953.

19. a pharmaceutical pack comprising an uPAR antagonist drug and instructions for administration of the drug to humans diagnostically tested for a single nucleotide polymorphism at one or more of positions: 14935, 15282, 19985, 20258, 33251, 36468, 36623 and 36720, each defined by the position of the reverse complement of EMBL Accession Number AC006953.

20. A computer readable medium having stored thereon a nucleic acid sequence comprising at least 17, preferably at least 20 consecutive bases of the uPAR gene sequence, which sequence includes at least one of the polymorphisms at positions: 14935, 15282, 19985, 20258, 33251, 36468, 36623 and 36720, according to the position of the reverse complement of EMBL Accession Number AC006953.

21. A computer readable medium having stored thereon a nucleic acid sequence comprising at least 17, preferably at least 20 consecutive bases of the uPAR gene sequence, which sequence includes at least one of the following polymorphisms: G at position 14935, A at position 15282, C at position 19985, A at position 20258, G at position 33251, T at position 36468, C at position 36623 and A at position 36720, or a complementary strand thereof.

22. A computer readable medium having stored thereon a nucleic acid sequence comprising any of the sequences of SEQ ID No. 15 to SEQ ID No. 22, or a complementary sequence thereto.
